## Europäisches Patentamt
## European Patent Office
## Office européen des brevets

(19)

(11) Veröffentlichungsnummer: **0 043 055**
**A1**

(12)

# EUROPÄISCHE PATENTANMELDUNG

(21) Anmeldenummer: 81104751.3

(22) Anmeldetag: 22.06.81

(51) Int. Cl.³: **A 61 K 31/635**
**//(A61K31/635, 31/505)**

(30) Priorität: 26.06.80 CH 4916/80

(43) Veröffentlichungstag der Anmeldung:
06.01.82 Patentblatt 82/1

(84) Benannte Vertragsstaaten:
BE CH DE FR GB IT LI LU NL SE

(71) Anmelder: F. HOFFMANN-LA ROCHE & CO.
Aktiengesellschaft

CH-4002 Basel(CH)

(72) Erfinder: Böhni, Erika, Dr.
Clarahofweg 38
CH-4058 Basel(CH)

(72) Erfinder: Plozza-Nottebrock, Helene, Dr.
Hirschgässlein 40
CH-4051 Basel(CH)

(72) Erfinder: Then, Rudolf, Dr.
Schutzackerstrasse 39
D-7858 Weil/Rhein(DE)

(74) Vertreter: Lederer, Franz, Dr. et al,
Patentanwälte Lederer, Meyer Lucile-Grahn-Strasse 22
D-8000 München 80(DE)

(54) Wirkstoffkombinationen, deren Herstellung und Verwendung sowie Mittel auf deren Basis.

(57) Antibakterielle Wirkstoffkombinationen aus Sulfamera-zin und 2,4-Diamino-5-(4-brom-3,5-dimethoxybenzyl)-pyrimidin oder deren physiologisch verträglichen Salzen, deren Herstellung und Verwendung sowie diese enthaltende Mittel.

EP 0 043 055 A1

F.Hoffmann-La Roche & Co. Aktiengesellschaft,Basel,Schweiz

0043055

2 2 Juni 1981

RAN 4440/157

Wirkstoffkombinationen,
deren Herstellung und Verwendung sowie Mittel auf deren Basis

Die vorliegende Erfindung betrifft neue Wirkstoffkombinationen und zwar Kombinationen des Sulfonamids Sulfamerazin (2-Sulfanilamido-4-methylpyrimidin, SME) mit dem
Sulfonamidpotentiator 2,4-Diamino-5-(4-brom-3,5-dimethoxybenzyl)-pyrimidin (DBP) bzw. von deren physiologisch verträglichen Salzen. Die vorliegende Erfindung betrifft ferner
neue antibakteriell wirksame, pharmazeutische Mittel auf
der Basis dieser Wirkstoffkombinationen.

Sulfamerazin ist ein bekanntes Sulfonamid mit antibakterieller Wirkung (siehe z.B. Merck-Index, 9. Ausgabe,
1976, S. 1153). 2,4-Diamino-5-(4-brom-3,5-dimethoxybenzyl)-
pyrimidin ist ebenfalls eine bekannte Verbindung (siehe z.B.
deutsche Offenlegungsschrift Nr. 24 52 889). Es war ferner
bekannt, 2,4-Diamino-5-(4-brom-3,5-dimethoxybenzyl)-pyrimidin bzw. dessen Salze als antibakteriell wirksame Mittel
zu verwenden und mit antibakteriell wirksamen Sulfonamiden
zwecks gegenseitiger Potenzierung beider Aktivitäten zu
kombinieren. In der obengenannten deutschen Offenlegungsschrift sind bereits eine Anzahl von Sulfonamiden namentlich
aufgeführt, die nach damaligem Stand des Wissens als besonders geeignete Partner für derartige Kombinationen erschienen, z.B. Sulfadimethoxin (SDM) und Sulfamethoxazol (SMZ).

Mez 23.2.81

0043055

Neuere Untersuchungen haben nun ergeben, dass das bisher als Kombinationspartner für 2,4-Diamino-5-(4-brom-3,5-dimethoxybenzyl)-pyrimidin nicht in Betracht gezogene und als solches nicht genannte Sulfamerazin sich besonders gut für eine derartige Kombination eignet. Die erfindungsgemässen neuen Wirkstoffkombinationen weisen gegenüber bereits bekannten Wirkstoffkombinationen gleicher Wirkungsrichtung therapeutische Vorteile auf, die für den Fachmann nicht vorhersehbar waren und die beispielsweise eine niedrigere Dosierung als bei ähnlichen Kombinationspräparaten üblich, ermöglichen.

In der folgenden Tabelle sind die in in-vivo-Vergleichsversuchen ermittelten $CD_{50}$-Werte (mg/kg) bei verschiedenen Infektionen mit pathogenen Mikroorganismen für folgende Wirkstoffkombinationen dargestellt:

SME/DBP = erfindungsgemässe Kombination

SDM/DBP = sich aus dem Stand der Technik ergebende Kombination

SMZ/TMP = bekannte Kombination (TMP = Trimethoprim)

## Tabelle

| Infektion | Gew.-Verh. | $CD_{50}$ [mg/kg]  p.o. | | |
|---|---|---|---|---|
| | | SME/DBP | SDM/DBP | SMZ/TMP |
| E. coli - Septikämie (Maus) | 1 : 1 | 4.0 | 8.1 | 4.8 |
| | 2 : 1 | 5.4 | 5.4 | 5.8 |
| | 5 : 1 | 3.6 | 8.6 | 4.2 |
| Klebsiellen-Septikämie (Maus) | 1 : 1 | 2.4 | 7.2 | 2.5 |
| | 2 : 1 | 1.35 | 4.5 | 1.5 |
| | 5 : 1 | 2.1 | 4.8 | 2.7 |
| Proteus-Septikämie (Maus) | 1 : 1 | 3.2 | 5.8 | 2.7 |
| | 2 : 1 | 1.95 | 4.35 | — |
| | 5 : 1 | 3.0 | 7.2 | 1.8 |
| Salmonellen-Septikämie (Maus) | 1 : 1 | 40.2 | — | 278.0 |
| | 2 : 1 | 23.4 | — | 187.5 |
| | 5 : 1 | 50.4 | — | 403.8 |
| Staphylokokken-Nephritis (Ratte) | 1 : 1 | 2.2 | — | 5.8 |
| | 2 : 1 | 1.8 | — | 5.1 |

0043055

Physiologisch verträgliche Salze bildet DBP mit den üblicherweise für diesen Zweck in Frage kommenden anorganischen und organischen Säuren wie Salzsäure, Schwefelsäure, Phosphorsäure, Ameisensäure, Essigsäure, Bernsteinsäure, Milchsäure, Citronensäure, Maleinsäure, Fumarsäure, Weinsäure, Methansulfonsäure, p-Toluolsulfonsäure etc. Als physiologisch verträgliche Salze des Sulfamerazins kommen vor allem die Alkali-, Erdalkali-und (gegebenenfalls substituierten) Ammoniumsalze in Frage.

Das Gewichtsverhältnis von Sulfonamid zu Potentiator kann in den erfindungsgemässen Wirkstoffkombinationen und pharmazeutischen Präparaten im Bereich von 10:1 bis 1:1 variieren und liegt vorzugsweise bei 5:1.

Die Herstellung der pharmazeutischen Präparate kann in jedem Fachmann geläufiger Weise durch Vermischen der Wirkstoffkomponenten mit geeigneten, nicht-toxischen, inerten, therapeutisch verträglichen festen oder flüssigen Trägermaterialien, vorzugsweise Feststoffen, einschliesslich der üblichen Hilfsmittel, wie Stabilisierungs-, Konservierungs-, Netz- oder Emulgiermitteln, Mitteln zur geschmacklichen Verbesserung, Salzen zur Veränderung des osmotischen Druckes oder Puffersubstanzen, erfolgen.

Die Applikation kann parenteral oder, vorzugsweise, oral erfolgen. Als Dosierungseinheit bei Tabletten empfehlen sich die im folgenden Beispiel angegebenen Mengen. Bei der Behandlung werden zweckmässigerweise unter Berücksichtigung der Art und Stärke der vorliegenden Infektion, Dosen verabreicht, die geringer sind als die analoger Präparate. Bei Erwachsenen ist eine geeignete Initialdosis beispielsweise 1000 mg Sulfamerazin + 200 mg DBP und die Aufrechterhaltungsdosis 500 mg Sulfamerazin + 100 mg DBP (alle 24 Stunden), bei einer Therapiedauer von wenigen Tagen bis zu etwa 2 Wochen oder auch länger.

<u>Beispiel</u>

Es wurden in an sich bekannter Weise Tabletten hergestellt, enthaltend pro Stück (mg):

| | | | | |
|---|---|---|---|---|
| SME | 1000,00 | 500,00 | 500,00 | 125,00 |
| DBP | 100,00 | 100,00 | 125,00 | 125,00 |
| Polyvinylpyrrolidon | 25,00 | 12,50 | 12,50 | 12,50 |
| Na-Carboxymethylstärke | 15,00 | 12,50 | 12,50 | 12,50 |
| Dioctylnatrium-sulfosuccinat | 0,30 | 0,15 | 0,15 | 0,15 |
| Magnesiumstearat | 19,70 | 9,85 | 9,85 | 9,85 |
| | 1170,00 | 635,00 | 660,00 | 265,00 |

22. Juni 1981
0043055
DS RAN 4440/157

## Patentansprüche

1. Wirkstoffkombinationen von Sulfamerazin und 2,4-Diamino-5-(4-brom-3,5-dimethoxybenzyl)-pyrimidin oder deren physiologisch verträglichen Salzen.

2. Wirkstoffkombinationen gemäss Anspruch 1, gekennzeichnet durch ein Gewichtsverhältnis von 10:1-1:1 (Sulfonamid/Potentiator).

3. Wirkstoffkombinationen gemäss Anspruch 1 oder 2, gekennzeichnet durch ein Gewichtsverhältnis von 5:1 (Sulfonamid/Potentiator).

4. Antibakterielle Mittel auf der Basis einer Wirkstoffkombination gemäss den Ansprüchen 1-3.

5. Verfahren zur Herstellung antibakterieller Mittel auf der Basis einer Wirkstoffkombination gemäss den Ansprüchen 1-3, dadurch gekennzeichnet, dass die Wirkstoffe mit nicht-toxischen, inerten, therapeutisch verträglichen Trägermaterialien vermischt und gegebenenfalls in eine geeignete galenische Form gebracht werden.

6. Verwendung der Wirkstoffkombinationen gemäss den Ansprüchen 1-3 bei der Prophylaxe und Therapie bakterieller Infektionen.

***

Nummer der Anmeldung

**EUROPÄISCHER TEILRECHERCHENBERICHT,**
der nach Regel 45 des Europäischen Patentübereinkommens für das weitere Verfahren als
europäischer Recherchenbericht gilt

Europäisches
Patentamt

EP 81 10 4751

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X/D | DE - A - 2 452 889 (F. HOFFMANN-LA ROCHE & Co. A.G.)<br><br>* Seiten 26,27; Ansprüche 4-9; Seite 8, Abschnitte 2,3 *<br><br>--- | 1-5 |
| | UNLISTED DRUGS, Band 28, November 1976,Heft 11, CHATHAM, N.J. (US)<br><br>* Seite 190e: "BERLOCOMBIN" *<br><br>--- | 1-5 |
| A | MARTIN NEGWER: "Organisch-chemische Arzneimittel und ihre Synonyma", 1978, AKADEMIE-VERLAG, Band 1, BERLIN (DE)<br><br>* Seite 289, Nr. 1592: "Sulfamera-<br><br>--------- | 1-5 |

**KLASSIFIKATION DER ANMELDUNG (Int. Cl.³)**

A 61 K 31/635//
(A 61 K 31/635
31/505)

**RECHERCHIERTE SACHGEBIETE (Int. Cl.³)**

A 61 K 31/635
A 61 K 31/505
C 07 D 239/48

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5.

Unvollständig recherchierte Patentansprüche:

Nicht recherchierte Patentansprüche: 6: Verfahren zur chirurgischen oder

Grund für die Beschränkung der Recherche: therapeutischen Behandlung des menschlichen oder tierischen Körpers (siehe Art. 52(4) des Europäischen Patentübereinkommens).

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&. Mitglied der gleichen Patentfamilie, übereinstimmendes Dokument

| Recherchenort | Bdatum der Recherche | Prüfer |
|---|---|---|
| Den Haag | 22.09.1981 | BRINKMANN |

EPA Form 1505.1   06.78